(19) Europäisches Patentamt
European Patent Office
Office européen des brevets

(11) **EP 2 605 002 B1**

(12) **FASCICULE DE BREVET EUROPEEN**

(45) Date de publication et mention
de la délivrance du brevet:
**02.01.2019 Bulletin 2019/01**

(51) Int Cl.:
*G01N 21/47* *(2006.01)*    *G01N 21/49* *(2006.01)*
*G01N 21/64* *(2006.01)*    *A61B 5/00* *(2006.01)*
*G01N 21/17* *(2006.01)*    *G09B 23/28* *(2006.01)*

(21) Numéro de dépôt: **12197003.2**

(22) Date de dépôt: **13.12.2012**

(54) **Procédé de reconstruction de propriétés optiques d'un milieu diffusant à l'aide d'une combinaison d'une pluralité de transformées de Mellin-Laplace d'une grandeur comportant une distribution temporelle d'un signal reçu, et système de reconstruction associé**

Verfahren zur Rekonstruktion der optischen Eigenschaften eines streuenden Mediums unter Verwendung einer Kombination aus einer Vielzahl von Mellin-Laplace Transformationen einer Größe, welche auf einer zeitlichen Verteilung von einem empfangenen Signal basiert, und zugeordnetes Rekonstruktionssystem

Method for reconstructing the optical properties of a scattering medium using a combination of a plurality of Mellin-Laplace transforms of a magnitude comprising a time distribution of a received signal, and associated reconstruction system

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.12.2011 FR 1161832**

(43) Date de publication de la demande:
**19.06.2013 Bulletin 2013/25**

(73) Titulaire: **Commissariat à l'Énergie Atomique et aux Énergies Alternatives**
**75015 Paris (FR)**

(72) Inventeur: **Herve, Lionel**
**38700 Corenc (FR)**

(74) Mandataire: **Lavoix**
**2, place d'Estienne d'Orves**
**75441 Paris Cedex 09 (FR)**

(56) Documents cités:
**EP-A1- 1 884 765    US-A1- 2008 260 647**

- **NICOLAS DUCROS ET AL: "A comprehensive study of the use of temporal moments in time-resolved diffuse optical tomography: part II. Three-dimensional reconstructions; Reconstructions from moments in time-resolved diffuse optical tomography", PHYSICS IN MEDICINE AND BIOLOGY, TAYLOR AND FRANCIS LTD. LONDON, GB, vol. 54, no. 23, 7 décembre 2009 (2009-12-07), pages 7107-7119, XP020167032, ISSN: 0031-9155**
- **ARRIDGE S R: "Optical tomography in medical imaging", INVERSE PROBLEMS, INSTITUTE OF PHYSICS PUBLISHING, BRISTOL, GB, no. 15, 1 janvier 1999 (1999-01-01), pages R41-R93, XP002404811, ISSN: 0266-5611**
- **PUSZKA A ET AL: "Time-Resolved Reflectance DOT: Experimental Results for Imaging Absorption Contrast in Depth", BIOMEDICAL OPTICS (BIOMED) 2012 PAPER: BW1A.2, 2012, XP002676624,**

**Description**

La présente invention concerne un procédé de reconstruction de propriétés optiques d'un milieu diffusant à l'aide d'un système de reconstruction comportant au moins une source de rayonnement propre à éclairer un milieu et au moins un détecteur propre à recevoir un signal émis par le milieu, le procédé comprenant:

- l'éclairement du milieu par la ou chaque source de rayonnement,
- la réception, par le ou chaque détecteur, d'un signal émis par le milieu, et
- l'élaboration, pour au moins un couple source-détecteur, d'une première distribution du signal reçu par le détecteur correspondant,
- le calcul de la transformée de Mellin-Laplace, pour un ordre et une variable donnés, d'une grandeur comportant la première distribution, l'ordre étant un nombre entier, la variable étant un nombre réel, et
- la reconstruction de propriétés optiques du milieu à l'aide de la transformée de Mellin-Laplace de ladite grandeur.

L'invention concerne également un tel système de reconstruction.

L'invention se situe notamment dans le domaine de l'instrumentation médicale, et en particulier dans le domaine de l'imagerie optique de diffusion appliquée à des tissus biologiques.

L'invention s'applique à la détection de différentes pathologies, telles que des cancers du sein, de la prostate ou encore du tube digestif, sein ou cerveau. L'invention s'applique également à la dermatologie, notamment à la caractérisation d'une réaction d'inflammation cutanée.

EP 1 884 765 A1 décrit un procédé de reconstruction de propriétés optiques d'un milieu, à l'aide d'un système de reconstruction comportant une source de rayonnement propre à éclairer le milieu et un détecteur propre à recevoir un signal émis par le milieu. Le procédé de reconstruction comprend l'éclairement du milieu par la source, la réception, par le détecteur, d'un signal émis par le milieu, et l'élaboration pour le couple source - détecteur d'une première distribution du signal reçu par le détecteur, à savoir le signal de fluorescence. EP 1 884 765 A1 décrit également le calcul d'une transformée de Mellin-Laplace des signaux de fluorescence ou le calcul des moments d'ordres supérieurs des fonctions de fluorescence normalisées.

On connaît l'article « Optical tomography in medical imaging » de S.R. Arridge publiée dans la revue « Inverse Problems » en 1999, citant l'utilisation possible d'une transformée de Mellin-Laplace à l'ordre n pour des applications en tomographie optique.

Cependant, cet article ne détaille pas précisément comment cette transformée peut être mise en oeuvre dans le cadre de la reconstruction des propriétés optiques d'un milieu diffusant, avec les avantages décrits ci-après.

Le but de l'invention est donc de proposer un procédé de reconstruction des propriétés optiques d'un milieu diffusant ainsi qu'un système de reconstruction utilisant avantageusement la transformée de Mellin Laplace, permettant notamment l'accès à des propriétés optiques de diffusion usuellement difficiles à établir, et par exemple la distribution spatiale des coefficients d'absorption ou de diffusion d'un milieu.

A cet effet, l'invention a pour objet un procédé de reconstruction du type précité, caractérisé en ce que l'étape de calcul comporte le calcul d'une pluralité de transformées de Mellin-Laplace de ladite grandeur pour des valeurs distinctes de l'ordre, et en ce que l'étape de reconstruction est effectuée à partir d'une combinaison de la pluralité de transformées de Mellin-Laplace.

Par reconstruction des propriétés optiques, on entend par exemple :

- la reconstruction des propriétés d'absorption, ces dernières étant notamment caractérisées par le coefficient d'absorption, noté $\mu_a$,
- la reconstruction des propriétés de diffusion, ces dernières étant notamment caractérisées par le coefficient de diffusion réduit $\mu'_s$ ou par le coefficient de diffusion D, et
- la reconstruction des propriétés de fluorescence, ces dernières étant notamment caractérisées par une fonction de réponse (F) d'un fluorophore, ou par une concentration c d'un fluorophore, ou encore par toute autre grandeur exprimant une quantité q d'un fluorophore, ce dernier étant par exemple endogène ou exogène.

Suivant d'autres aspects avantageux de l'invention, le procédé de reconstruction comporte une ou plusieurs des caractéristiques suivantes, prises isolément ou suivant toutes les combinaisons techniquement possibles :

- l'étape de calcul comporte le calcul d'au moins une transformée de Mellin-Laplace de ladite grandeur pour une valeur de l'ordre supérieure ou égale à 5, de préférence supérieure ou égale à 8,
- l'étape de calcul comporte le calcul de $n_{max}+1$ transformées de Mellin-Laplace, l'ordre prenant successivement toutes les valeurs comprises entre 0 et $n_{max}$, avec $n_{max}$ supérieur ou égal à 5, de préférence supérieur ou égal à 8,
- la valeur de la variable est comprise entre 1 ns$^{-1}$ et 20 ns$^{-1}$,

- le procédé comprend en outre :

  + la détermination, pour au moins un couple source-détecteur, d'une première fonction de modélisation d'un signal de diffusion de la lumière entre la source et le détecteur dans le milieu,
  + l'élaboration, pour ledit au moins un couple source-détecteur, d'une deuxième distribution d'un signal reçu par le détecteur pour un milieu de référence, le signal reçu étant émis par le milieu de référence suite à l'éclairement dudit milieu par la source, et
  + la détermination, pour ledit au moins un couple source-détecteur, d'une deuxième fonction de modélisation d'un signal de diffusion de la lumière entre la source et le détecteur dans le milieu de référence,
  et dans lequel ladite grandeur dépend de la première distribution, de la première fonction de modélisation, de la deuxième distribution et de la deuxième fonction de modélisation,

- ladite grandeur est obtenue par soustraction du produit de la deuxième distribution et de la première fonction de modélisation du produit de la première distribution et de la deuxième fonction de modélisation,
- ladite grandeur est le ratio entre le produit de la première distribution et de la deuxième fonction de modélisation et le produit de la deuxième distribution et de la première fonction de modélisation,
- la ou chaque source de rayonnement comporte une source de lumière puisée,
- le ou chaque détecteur est un détecteur résolu en temps, et
- les propriétés optiques comprennent au moins un élément parmi le groupe consistant en :

  + les propriétés d'absorption de la lumière, caractérisées notamment par le coefficient d'absorption,
  + les propriétés de diffusion, caractérisées notamment par le coefficient de diffusion réduit ou par le coefficient de diffusion, et
  + les propriétés de fluorescence, caractérisées notamment par une fonction de réponse d'un fluorophore, ou par une concentration du fluorophore, ou encore par une grandeur fonction d'une quantité du fluorophore. L'invention a également pour objet un système de reconstruction comprenant

- au moins une source de rayonnement propre à éclairer le milieu,
- au moins un détecteur propre à recevoir un signal émis par le milieu,
- des moyens d'élaboration, pour au moins un couple source-détecteur, d'une distribution du signal reçu par le détecteur correspondant,
- des moyens de calcul de la transformée de Mellin-Laplace, pour un ordre et une variable donnés, d'une grandeur comportant la distribution, l'ordre étant un nombre entier, la variable étant un nombre réel, et
- des moyens de reconstruction de propriétés optiques du milieu à l'aide de la transformée de Mellin-Laplace de ladite grandeur,

caractérisé en ce que les moyens de calcul sont propres à calculer une pluralité de transformées de Mellin-Laplace de ladite grandeur pour des valeurs distinctes de l'ordre, et en ce que les moyens de reconstruction sont propres à reconstruire les propriétés optiques du milieu à partir d'une combinaison de la pluralité de transformées de Mellin-Laplace.

Suivant un autre aspect avantageux de l'invention, le système de reconstruction comprend en outre des moyens de calcul propres à calculer au moins une transformée de Mellin-Laplace de ladite grandeur pour une valeur de l'ordre supérieure ou égale à 5, de préférence supérieure ou égale à 8.

Ces caractéristiques et avantages de l'invention apparaîtront à la lecture de la description qui va suivre, donnée uniquement à titre d'exemple, et faite en référence aux dessins annexés sur lesquels :

- la figure 1 est une représentation schématique d'un système de reconstruction selon l'invention, propre à reconstruire une image d'un milieu,
- la figure 2 est un ensemble de courbes représentant une distribution temporelle d'un signal reçu par le système de la figure 1 et les transformées de Mellin-Laplace de cette distribution temporelle calculées pour des valeurs d'ordre comprises entre 0 et 9,
- la figure 3 est un organigramme d'un procédé de reconstruction selon l'invention,
- la figure 4 est un ensemble d'images représentant des cartes de sensibilité pour les transformées de Mellin-Laplace calculées, chaque image correspondant à une valeur d'ordre respective et comprise entre 0 et 8,
- la figure 5 est une vue d'une image du milieu reconstruite avec un système de reconstruction et un procédé de reconstruction de l'état de la technique, et
- la figure 6 est une vue analogue à celle de la figure 5 avec un système de reconstruction et un procédé de reconstruction selon l'invention.

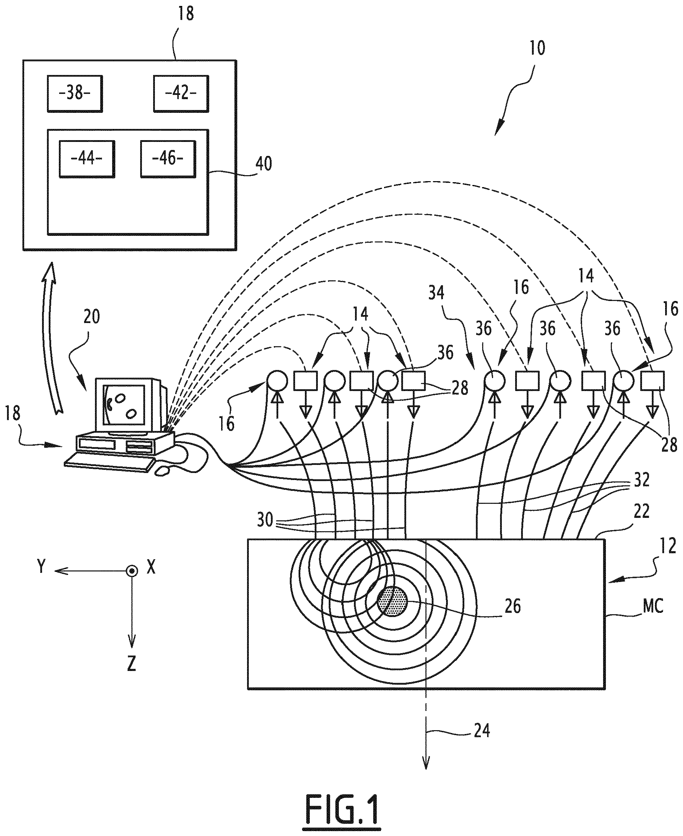

Sur la figure 1, un système de reconstruction 10 est destiné à réaliser un examen d'un milieu 12 via l'acquisition d'une image du milieu 12, puis le traitement de cette image.

Le système de reconstruction 10 comprend une pluralité de sources de rayonnement 14 aptes à éclairer le milieu 12 et une pluralité de détecteurs 16 propres à recevoir un signal émis par le milieu 12. Les sources de rayonnement 14 et les détecteurs 16, sont, par exemple, agencés dans une sonde, non représentée.

Le système 10 comprend une unité de traitement d'informations 18 et un écran 20 d'affichage d'une image du milieu.

Dans le mode de réalisation décrit, le système 10 est un système temporel, et chaque source de rayonnement 14 est une source de rayonnement puisée. Chaque détecteur 16 est un détecteur résolu en temps, c'est-à-dire un détecteur permettant de mesurer la distribution temporelle du temps d'arrivée des photons, également appelés TCSPC (de l'anglais *Time-Correlated Single Photon Counting).*

Selon l'exemple représenté, le milieu 12 présente une surface d'observation 22, en forme d'un plan parallèle à un axe longitudinal X et un axe transversal Y, et au contact de laquelle les sources 14 et les détecteurs 16 sont propres à être appliqués. Le milieu 12 présente une direction d'observation 24 s'étendant selon un axe vertical Z et sensiblement perpendiculairement à la surface d'observation 22.

Dans l'exemple de réalisation de la figure 1, le milieu 12 est un milieu à caractériser, noté MC et visible sur la figure 1. Le milieu à caractériser MC comporte des tissus biologiques d'un animal ou de l'homme. Le milieu à caractériser MC est, par exemple, une zone d'un organe tel que le cerveau, un sein, la prostate, le tube digestif ou encore d'autres organes dans lesquels des fluorophores sont susceptibles d'être injectés. Plus généralement, le milieu à caractériser MC est un milieu biologique, par exemple un organe, dont on souhaite déterminer des propriétés optiques de diffusion et notamment la distribution spatiale de coefficients tels que le coefficient d'absorption ou le coefficient de diffusion.

Le milieu à caractériser MC est un milieu diffusant contenant des inclusions dont les propriétés optiques d'absorption ou de diffusion sont différentes de celles du milieu. 26, Une seule inclusion 26 est représentée sur la figure 1 pour la clarté du dessin.

Chaque source de rayonnement pulsée 14 comporte une source lumineuse pulsée 28 et une fibre optique d'excitation 30 reliée à la source pulsée 28 pour la transmission de l'impulsion lumineuse jusqu'au milieu 12. Lorsqu'une telle source fibrée est utilisée, on assimile l'extrémité libre de la fibre d'excitation 30 à la source s.

En variante non représentée, chaque source de rayonnement pulsée 14 comporte une fibre optique d'excitation 30 reliée à une unique source lumineuse pulsée commune à la pluralité de sources de rayonnement 14. Selon cette variante, le système 10 comprend en outre un commutateur optique ou un multiplexeur pour sélectionner la fibre d'excitation 30 dans laquelle le faisceau lumineux est envoyé.

En variante encore, l'ensemble des sources de rayonnement pulsée 14 est formé d'une unique source lumineuse pulsée et d'un dispositif à miroir de type MEMS (de l'anglais *MicroElectroMechanical Systems*), non représenté, pour balayer le milieu 12 avec la lumière issue de la source lumineuse puisée. En variante encore, la source 14 est une source multiplexée, délivrant des impulsions lumineuses à différentes longueur d'onde.

Dans le cas d'un examen de la surface 22 du milieu ou d'un examen à une faible profondeur du milieu 12, c'est-à-dire une profondeur de quelques millimètres à quelques centimètres, la longueur d'ondes de chaque source de rayonnement pulsée 14 est, de préférence, dans le domaine du visible ou du proche infrarouge, c'est-à-dire comprise entre 500 nm et 1300 nm. Le taux de répétition est d'environ 50 MHz, et en général compris entre 10 MHz et 100 MHz.

Les impulsions émises par chaque source de rayonnement pulsée 14 présentent une durée comprise entre 10 picosecondes et 1 nanoseconde, de préférence comprise 10 picosecondes et 100 picosecondes, chaque source lumineuse pulsée 28 étant propre à générer une impulsion de largeur temporelle de durée comprise entre 10 picosecondes et 1 nanoseconde, de préférence comprise 10 picosecondes et 100 picosecondes.

Chaque détecteur résolu en temps 16, noté également d, comporte une fibre optique de détection 32 reliée à un module de détection résolu en temps 34. Lorsqu'un tel détecteur fibré est utilisé, l'extrémité libre de cette fibre optique de détection 32 est assimilée au détecteur d.

Dans l'exemple de réalisation de la figure 1, le module de détection 34 comporte un organe de détection 36 pour chaque détecteur 16. En variante non représentée, le module de détection 34 comporte un organe de détection commun à plusieurs détecteurs 16, en particulier un unique organe de détection pour l'ensemble des détecteurs 16.

L'organe de détection 36 est, par exemple, un photomultiplicateur, ou une photodiode à avalanche, également appelée APD (de l'anglais *Avalanche Photodiode),* ou une photodiode à avalanche à photon unique, également SPAD (de l'anglais *Single-Photon Avalanche Diode)* ou encore un tube intensificateur d'images ayant une ou plusieurs anodes (de l'anglais *Multi-Channel Plate*).

La sonde est, dans cet exemple, une sonde compacte pour le diagnostic de certains cancers, tels qu'une sonde portative pour le diagnostic du cancer du sein, une sonde endorectale pour le diagnostic du cancer de la prostate, ou encore une sonde de dermatologie. En variante, la sonde est une sonde d'endoscope telle qu'une sonde souple pour le diagnostic du cancer digestif. En variante, la sonde est un dispositif applicable à d'autres organes.

L'unité de traitement de l'information 18 comporte un processeur de données 38 et une mémoire 40 associée au processeur 38.

L'unité de traitement 18 comporte des moyens 42 d'élaboration, pour au moins un couple source 14 - détecteur 16, d'une première distribution $B_{sd}$ d'un signal reçu par le détecteur 16 correspondant pour le milieu à caractériser MC, le signal reçu étant émis par le milieu diffusant à caractériser MC suite à l'éclairement dudit milieu MC par la source 14 correspondante. La source 14, et respectivement le détecteur 16, correspondants sont repérés par les indices s, et respectivement d.

Dans le mode de réalisation décrit, la première distribution est une distribution temporelle respectivement notée $B_{sd}(t)$. Les moyens d'élaboration 42 sont, de préférence, réalisés sous forme d'une ou plusieurs cartes électroniques connectées au détecteur 16 et permettant de mesurer la distribution temporelle du temps d'arrivée des photons.

Chaque source de lumière pulsée 28 comporte un laser impulsionnel. En variante, chaque source lumineuse pulsée 28 comporte une diode laser. En variante encore, chaque source lumineuse pulsée 28 comporte une source de lumière constante dont l'intensité lumineuse est modulée en des impulsions de durée équivalente par un dispositif à obturation rapide. Ainsi, la source est apte à émettre un rayonnement lumineux prenant la forme d'une impulsion temporelle.

Selon ce mode particulier, l'extrémité de chaque fibre d'excitation 30 s'étend perpendiculairement à la surface d'observation 22 c'est-à-dire selon l'axe Z, ou encore de manière oblique par rapport à l'axe Z afin d'émettre la lumière en biais par rapport à la surface d'observation 22.

La mémoire 40 est apte à stocker un logiciel 44 de calcul d'une pluralité de transformées de Mellin-Laplace $Y_{sd}^{(p,n)}$, pour une variable p, également appelée largeur, et des valeurs distinctes d'un ordre n donné, d'une grandeur $Y_{sd}(t)$ comportant la première distribution $B_{sd}(t)$, l'ordre n étant un nombre entier et la variable p étant un nombre réel.

La transformée de Mellin-Laplace d'ordre n et de largeur p d'une fonction f est notée $f^{(p,n)}$, et vérifie l'équation suivante :

$$f^{(p,n)} = \frac{1}{n!} \int_0^{+\infty} (pt)^n . \exp(-pt).f(t).dt \qquad (1)$$

où 1/n! représente un terme de normalisation.

La transformée de Mellin-Laplace $f^{(p,n)}$ de la fonction f s'écrit également :

$$f^{(p,n)} = \int_{-\infty}^{+\infty} W^{(p,n)}.f(t).dt \qquad (2)$$

où $w^{(p,n)}$ représente une fenêtre temporelle définie par l'équation suivante :

$$W^{(p,n)} = \frac{H(t).(pt)^n.\exp(-pt)}{n!} \qquad (3)$$

avec H(t) représentant la fonction connue de Heaviside.

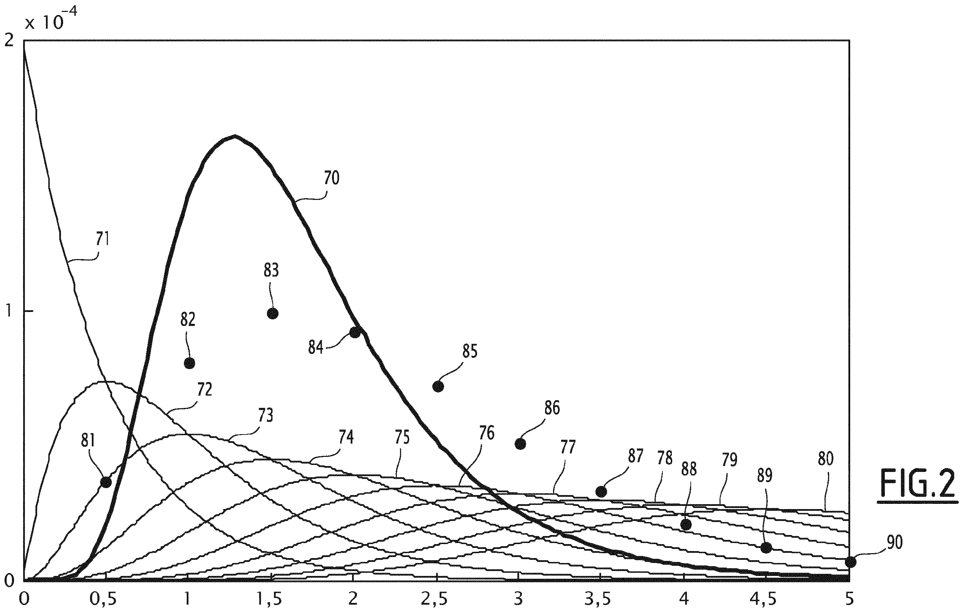

Sur la figure 2, la fonction f est représentée par la courbe 70 en trait gras, et les fenêtres temporelles $W^{(p,n)}$ sont représentées par les courbes 71 à 80, pour p égal à 2 ns$^{-1}$ et pour l'ordre n prenant respectivement les valeurs entières successives comprises entre 0 et $n_{max}$, avec $n_{max}$ égal à 9. Les positions $((n+1)/p, pf^{(p,n)})$ sont repérés par les points 81 à 90, pour les mêmes valeurs de la largeur p et de l'ordre n. L'aire des fenêtres temporelles $W^{(p,n)}$ est égale à 1/p, et la position moyenne de ces fenêtres temporelles $W^{(p,n)}$ est égale à (n+1)/p.

De manière générale, la transformée de Mellin-Laplace $f^{(p,n)}$ d''ordre n et de largeur p de la fonction f sera considérée comme toute fonction comprenant le terme $\int_0^{+\infty} (pt)^n.\exp(-pt).f(t).dt$ à un coefficient multiplicatif près.

Dans le mode de réalisation décrit, au moins une transformée de Mellin-Laplace $Y_{sd}^{(p,n)}$ est calculée pour une valeur de l'ordre n supérieure ou égale à 5, de préférence supérieure ou égale à 8.

Dans le mode de réalisation décrit, l'ordre n présente une valeur comprise entre 0 et une valeur maximale $n_{max}$. L'ordre n prend, par exemple, successivement toutes les valeurs comprises entre 0 et $n_{max}$. Autrement dit, $n_{max}+1$ transformées de Mellin-Laplace $Y_{sd}^{(p,n)}$ sont alors calculées par le logiciel de calcul 44. La valeur maximale $n_{max}$ est supérieure ou égale à 5, de préférence supérieure ou égale à 8.

La combinaison de transformées de Laplace d'ordre différents, et notamment des faibles ordres, c'est-à-dire n proche de 0, avec des ordres importants, c'est-à-dire n ≥ 5, permet la prise en compte de la contribution, au signal détecté, de photons émis à des instants très proches après l'impulsion d'excitation (ordres faibles) ainsi qu'à des instants plus éloignés (ordres importants), ces derniers étant appelés photons retardés, car ils sont détectés quelques ns après l'impulsion lumineuse d'excitation.

Dans le mode de réalisation décrit, la valeur de la largeur p est comprise entre 1 ns$^{-1}$ et 20 ns$^{-1}$, où ns est le symbole de la nanoseconde.

La mémoire 40 est également apte à stocker un logiciel 46 de reconstruction de propriétés optiques du milieu 12 à partir d'une combinaison de la pluralité de transformées de Mellin-Laplace $Y_{sd}^{(p,n)}$ de ladite grandeur $Y_{sd}(t)$. Le logiciel de reconstruction 46 est propre à reconstruire de la distribution spatiale de coefficients optiques, tels que le coefficient d'absorption optique $\mu_a$ ou le coefficient de diffusion D. En variante, le logiciel de reconstruction 46 est propre à reconstruire une carte de fluorescence du milieu 12.

Dans le mode de réalisation décrit, la grandeur $Y_{sd}(t)$ est un signal corrigé permettant de prendre en compte la réponse instrument, également appelé distribution corrigée, et est décrite plus en détail ci-après.

En variante, la grandeur sur laquelle est calculée la pluralité de transformées de Mellin-Laplace est la première distribution $B_{sd}(t)$.

En variante, les moyens de calcul 44 et les moyens de reconstruction 46 sont réalisés sous forme de composants logiques programmables ou encore sous forme de circuits intégrés dédiés.

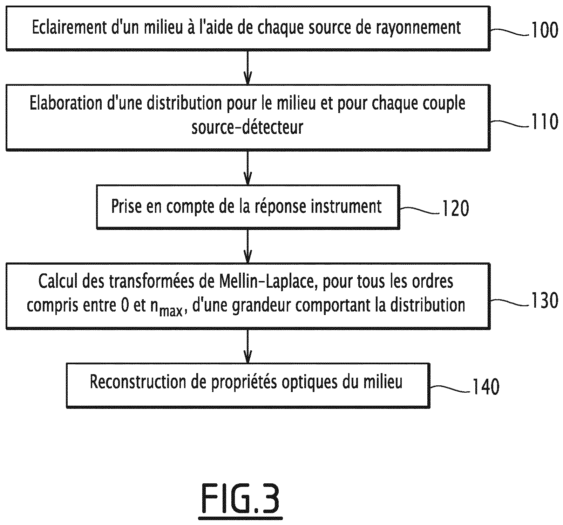

Le fonctionnement du système d'imagerie 10 selon l'invention est décrit ci-après à l'aide de la figure 3 représentant un organigramme du procédé de traitement d'image selon l'invention.

Lors de l'étape 100, le milieu à caractériser MC est éclairé par la ou les sources 14 correspondantes. En cas d'une pluralité de sources 14, l'éclairement du milieu 12 est effectué successivement pour chacune des sources 14. Lorsqu'il est éclairé, le milieu à caractériser MC émet en retour un signal optique de diffusion, susceptible d'être détecté par le ou chaque détecteur 16, et la première distribution du signal reçu $B_{sd}(t)$ est élaborée, lors de l'étape 110, pour chaque couple source 14 - détecteur 16, également noté (s, d), à l'aide des moyens d'élaboration 42.

La première distribution temporelle $B_{sd}(t)$ vérifie l'équation suivante :

$$B_{sd}(t) = IRF_{sd}(t) * G_{sd}(t) \qquad (4)$$

où $IRF_{sd}(t)$ représente la réponse instrument, c'est-à-dire l'influence de la source s et du détecteur d sur la première distribution élaborée, et

$G_{sd}(t)$ représente une première fonction de modélisation d'un signal de diffusion de la lumière entre la source 14 et le détecteur 16 dans le milieu à caractériser MC.

On note $S_s(t)$ la réponse temporelle de la source 14, également notée s. Dans cet exemple, la source 14 est une brève impulsion lumineuse, souvent modélisée par une distribution de type Dirac ponctuelle. $S_s(t)$ représente l'intensité temporelle du signal émis. Lorsque la source 14 est fibrée (source de lumière couplée à une fibre optique), c'est l'extrémité de la fibre optique d'excitation 30, qui est alors considérée comme la source, comme précédemment indiqué.

On note $D_d(t)$ la réponse temporelle du détecteur 16, également noté d. Cette réponse traduit notamment le délai entre l'arrivée d'un photon sur le détecteur 16 et sa détection effective. Lorsque le détecteur 16 est fibré (détecteur couplé à une fibre optique), c'est l'extrémité de la fibre de détection 32, qui est considérée comme le détecteur, comme précédemment indiqué.

On estime généralement la réponse instrument, notée $IRF_{sd}(t)$ l'indice sd corrrespondant à un couple source d'excitation s et détecteur d. Cette réponse instrument est obtenue en combinant Ss(t) et Dd(t) suivant : $IRF_{sd}(t) = S_s(t)*D_d(t)$, où * désigne le produit de convolution.

Lorsqu'on a plusieurs sources et plusieurs détecteurs, on détermine $IRF_{sd}(t)$ pour chaque couple source-détecteur, car chaque source et chaque détecteur a sa propre réponse. En pratique, cela se fait en plaçant une source (ou une extrémité de fibre optique constituant la source) en face d'un détecteur (ou d'une extrémité de fibre optique constituant le détecteur). Cela suppose un alignement soigné entre la source et le détecteur, et cela prend du temps, notamment lorsqu'on augmente le nombre de sources et de détecteurs. A titre d'exemple, 100 fonctions de réponse instrument $IRF_{sd}$ sont à déterminer lorsqu'on dispose de 10 sources et 10 détecteurs.

Lors de l'étape 120, la réponse instrument $IRF_{sd}(t)$ est prise en compte à l'aide d'une deuxième distribution temporelle $A_{sd}(t)$ et d'une deuxième fonction de modélisation $G_{sd}^0(t)$. La deuxième distribution temporelle $A_{sd}(t)$ est élaborée pour un milieu de référence MR et dans les mêmes conditions expérimentales que pour la première distribution temporelle $B_{sd}(t)$.

Le milieu de référence MR, également appelé fantôme, est connu en soi et est, par exemple, décrit dans le document FR 2 950 241 A1. Le fantôme MR a des caractéristiques optiques connues. Le fantôme MR est, par exemple, un fantôme ayant des coefficients d'absorption $\mu_a{}^0$ et de diffusion $D°$ de valeurs médianes pour les tissus humains, par exemple égales à 0,2 cm$^{-1}$, et respectivement à 10 cm$^{-1}$.

L'élaboration de la deuxième distribution temporelle $A_{sd}(t)$ est de préférence effectuée de manière préliminaire, avant l'étape 100, et le milieu de référence MR est alors remplacé par le milieu à caractériser MC au début de l'étape 100.

En variante, l'élaboration de la deuxième distribution temporelle $A_{sd}(t)$ est effectuée lors de l'étape 120, ce qui nécessite de changer le milieu 12, et de remplacer le milieu à caractériser MC par le milieu de référence MR.

La deuxième distribution temporelle $A_{sd}(t)$ vérifie alors l'équation suivante :

$$A_{sd}(t) = IRF_{sd}(t) * G_{sd}^0(t) \tag{5}$$

Les première et deuxième fonctions de modélisation $G_{sd}(t)$, $G_{sd}^0(t)$ sont, par exemple, des fonctions de Green, bien connues dans le domaine la diffusion optique. Chaque fonction de Green $G_{sd}(t)$, $G_{sd}^0(t)$ représente la densité de photons au niveau du détecteur 16 lorsque le milieu 12, c'est-à-dire le milieu de référence MR pour la première fonction de modélisation et respectivement le milieu à caractériser MC pour la deuxième fonction de modélisation, est éclairé par la source 14, où s et d sont les indices désignant respectivement la source 14 et le détecteur 16.

Le signal corrigé $Y_{sd}(t)$ est alors calculé en fonction de la première distribution $B_{sd}(t)$, de la première fonction de modélisation $G_{sd}(t)$, de la deuxième distribution $A_{sd}(t)$ et de la deuxième fonction de modélisation $G_{sd}^0(t)$.

Le signal corrigé $Y_{sd}(t)$ est le résultat d'une opération de comparaison du produit de la deuxième distribution $A_{sd}$ et de la première fonction de modélisation $G_{sd}$ avec le produit de la première distribution $B_{sd}$ et de la deuxième fonction de modélisation $G_{sd}^0$. L'opération de comparaison comporte une opération arithmétique du produit de la deuxième distribution $A_{sd}$ et de la première fonction de modélisation $G_{sd}$ avec le produit de la première distribution $B_{sd}$ et de la deuxième fonction de modélisation $G_{sd}^0$.

L'opération arithmétique est, par exemple, une soustraction du produit de la deuxième distribution $A_{sd}$ et de la première fonction de modélisation $G_{sd}$ du produit de la première distribution $B_{sd}$ et de la deuxième fonction de modélisation $G_{sd}^0$.

Dans le mode de réalisation décrit, le produit de la deuxième distribution $A_{sd}(t)$ et de la première fonction de modélisation $G_{sd}(t)$ et le produit de la première distribution $B_{sd}(t)$ et de la deuxième fonction de modélisation $G_{sd}^0(t)$ sont des produits de convolution, les première $B_{sd}(t)$ et deuxième $A_{sd}(t)$ distributions étant des distributions temporelles.

Le signal corrigé $Y_{sd}(t)$ vérifie l'équation suivante :

$$Y_{sd}(t) = B_{sd}(t) * G_{sd}^0(t) - A_{sd}(t) * G_{sd}(t) \tag{6}$$

En variante, l'opération arithmétique est un ratio entre le produit de la première distribution temporelle $B_{sd}(t)$ et de la deuxième fonction de modélisation $G_{sd}^0(t)$ et le produit de la deuxième distribution temporelle $A_{sd}(t)$ et de la première fonction de modélisation $G_{sd}(t)$.

Selon cette variante, le signal corrigé $Y_{sd}(t)$ vérifie alors l'équation suivante :

$$Y_{sd}(t) = \frac{B_{sd}(t) * G_{sd}^0(t)}{A_{sd}(t) * G_{sd}(t)} \tag{7}$$

En complément, la première fonction de modélisation $G_{sd}(t)$ est déterminée de manière approchée à l'aide de la fonction de Green $G_{sd}^1(t)$ pour le milieu à caractériser MC, et on obtient alors les équations suivantes :

$$
\begin{aligned}
Y_{sd}(t) &= B_{sd}(t) * G_{sd}^0(t) - A_{sd}(t) * G_{sd}^1(t) \\
&= IRF_{sd}(t) * G_{sd}(t) * G_{sd}^0(t) - IRF_{sd}(t) * G_{sd}^0(t) * G_{sd}^1(t) \\
&= IRF_{sd}(t) * G_{sd}^0(t) * \left( G_{sd}(t) - G_{sd}^1(t) \right) \\
&= A_{sd}(t) * \left( G_{sd}(t) - G_{sd}^1(t) \right) \\
&= -A_{sd}(t) * \left( \int G_s^1(\vec{r},t) * \delta\mu_a(\vec{r},t) * G_d^1(\vec{r},t)d\vec{r} + \int \vec{\nabla}G_s^1(\vec{r},t) * \delta D(\vec{r},t) * \vec{\nabla}G_d^1(\vec{r},t)d\vec{r} \right)
\end{aligned} \tag{8}
$$

où $\mu_a$ et D représentent respectivement le coefficient d'absorption et le coefficient de diffusion du milieu à caractériser MC.

IRF$_{sd}$(t) représente la réponse instrument,

G$_s$(r,t) = G(r$_s$,r,t) est la fonction de Green représentant la densité de photons à un endroit r du milieu MC lorsque le milieu MC est éclairé par la source s située en r$_s$,

G$_d$(r,t) = G(r$_d$,r,t) est la fonction de Green représentant la densité de photons à l'endroit r du milieu MC lorsque le milieu à caractériser MC est éclairé par la source s située en r$_d$. On écrit également G$_d$(r,t) = G(r,r$_d$,t). Ainsi, cette fonction de Green représente également la densité de photons au niveau du détecteur (r$_d$) lorsque le milieu à caractériser MC est éclairé par une source située en r.

Autrement dit, G$_{sd}$(t) correspond aux vraies valeurs $\mu_a$(r, t) et D(r, t), c'est-à-dire aux valeurs recherchées, et G$_{sd}^1$(t) correspond à des valeurs approchées $\mu_a^1$(r, t) et D$^1$(r, t), avec $\mu_a$(r, t) = $\mu_a^1$(r, t) + $\delta\mu_a$(r, t) et D(r, t) = D$^1$(r, t) + $\delta$D(r, t). Le processus de reconstruction des propriétés optiques est généralement itératif, de telle sorte qu'au cours de chaque itération, on obtient des valeurs $\mu_a^1$(r) et D$^1$(r), auxquelles correspondent des fonctions de Green $G_{sd}^1(t)$ pour chaque couple source-détecteur sd. L'objectif du procédé de reconstruction est alors de minimiser $\delta\mu_a$(r, t) et $\delta$D(r, t), ce qui permet de faire tendre $\mu_a^1$(r, t) et D$^1$(r, t) respectivement vers $\mu_a$(r, t) et D(r, t).

Les coefficients $\mu_a$ et D sont constants dans le temps, et les valeurs $\mu_a$(r, t) = $\mu_a$(r)$\partial$(t) et D(r,t) = D(r)$\partial$(t), $\partial$(t) représentent une distribution de Dirac à l'instant t.

Le calcul du signal corrigé Y$_{sd}$(t), également appelé distribution temporelle corrigée, s'affranchit donc de la connaissance précise de la réponse instrument IRF$_{sd}$(t) d'après les équations (6), (7) ou encore (8). Ceci permet d'éviter des mesures expérimentales relativement longues et fastidieuses afin de déterminer la réponse instrument du système d'imagerie 10.

En variante, la réponse instrument IRF$_{sd}$(t), représentant la répartition temporelle de l'impulsion générée par la source de rayonnement et détectée par le détecteur en l'absence de milieu diffusant, est mesurée, pour chaque couple source-détecteur (s, d), lors d'une opération de calibrage au cours de laquelle la source s est placée face au détecteur d en l'absence du milieu 12.

Lors de l'étape 130, les transformées de Mellin-Laplace Y$_{sd}^{(p,n)}$ de la distribution corrigée sont alors calculées pour différentes valeurs de l'ordre n comprises entre 0 et n$_{max}$, de préférence pour toutes les valeurs successives comprises entre 0 et n$_{max}$, suivant l'équation suivante :

$$Y_{sd}^{(p,n)} = \sum_{i+j=n}\left(B_{sd}^{(p,i)}.G_{sd}^{0(p,j)} - A_{sd}^{(p,i)}.G_{sd}^{(p,j)}\right) \tag{9}$$

Or, la distribution corrigée Y$_{sd}$(t) vérifie l'équation suivante :

$$Y_{sd}(t) = -A_{sd}(t)*\left(\int G_s^1(\vec{r},t)*\delta\mu_a(\vec{r},t)*G_d^1(\vec{r},t)d\vec{r} + \int \vec{\nabla}G_s^1(\vec{r},t)*\delta D(\vec{r},t)*\vec{\nabla}G_d^1(\vec{r},t)d\vec{r}\right) \tag{10}$$

Les équations (9) et (10) permettent alors d'obtenir l'équation suivante, étant donné que la transformée de Mellin-Laplace d'un produit de convolution de deux termes est égale au produit des transformées de Mellin-Laplace de chacun de ces deux termes :

$$Y_{sd}^{(p,n)} = -\sum_{i+j+k=n}A_{sd}^{(p,k)}.\left(\int G_s^{1(p,i)}(\vec{r}).\delta\mu_a(\vec{r}).G_d^{1(p,j)}(\vec{r})d\vec{r} + \int \vec{\nabla}G_s^{1(p,i)}(\vec{r}).\delta D(\vec{r}).\vec{\nabla}G_d^{1(p,j)}(\vec{r})d\vec{r}\right) \tag{11}$$

L'équation (11) permet ainsi de calculer la transformée de Mellin-Laplace à l'ordre n de la distribution corrigée Y$_{sd}^{(p,n)}$ des transformées de Mellin-Laplace de la deuxième distribution temporelle A$_{sd}$(t) et des fonctions de Green G$_s^1$(r), G$_d^1$(r) approchant la première fonction de modélisation G$_{sd}$(t).

Le milieu 12 est discrétisé en une pluralité M de voxels référencés m, avec m compris entre 1 et M, afin de calculer les transformées de Mellin-Laplace des fonctions de Green G$_s^1$(r), G$_d^1$(r). Les fonctions de Green G$_s^1$(r), G$_d^1$(r) discrétisées pour chaque voxel m sont notées G$_s^1$(r$_m$), G$_d^1$(r$_m$).

Selon la variante dans laquelle la réponse instrument IRF$_{sd}$(t) est mesurée pour chaque couple source-détecteur (s, d), lors d'une opération de calibrage, les transformées de Mellin-Laplace de la première distribution B$_{sd}^{(p,n)}$ et de la réponse instrument IRF$_{sd}^{(p,n)}$ sont calculées pour différentes valeurs de l'ordre n comprises entre 0 et n$_{max}$, de préférence

pour toutes les valeurs successives comprises entre 0 et $n_{max}$.

La reconstruction de propriétés optiques du milieu 12 est alors effectuée lors de l'étape 140, afin de déterminer par exemple les vecteurs $\vec{\mu_a}$ et $\vec{D}$, dont les termes $\mu_a(m)$ et D(m), discrétisés pour chaque voxel m, c'est-à-dire $\mu_a$ ($\vec{r} = \vec{r}_m$) et $D(\vec{r} = \vec{r}_m)$, constituent les cartes des propriétés optiques recherchées.

L'étape de reconstruction vise alors à résoudre le système matriciel suivant :

$$\underline{Y} = \underline{W}\,\underline{X} \,, \tag{12}$$

où $\underline{Y}$ représente un vecteur des observations, $\underline{W}$ représente une matrice de passage, et $\underline{X}$ représente un vecteur des inconnues.

Le vecteur des observations $\underline{Y}$ contient les Nn transformées de Mellin-Laplace de la distribution corrigée $Y_{sd}^{(p,n)}$, avec Nn égal à $n_{max}+1$, calculées pour toutes les valeurs successives de l'ordre n comprises entre 0 et $n_{max}$ et pour les couples source-détecteur (s, d) considérés.

On définit, pour chaque triplet (s, d, n) des indices s, d et de l'ordre n, un indice I de la manière suivante :

$$I = (s-1)\times Nd \times Nn + (d-1)\times Nn + n \tag{13}$$

où Nd est le nombre de détecteurs 16 considérés.

La valeur maximale de l'indice I est égale à Imax, tel que :

$$I_{max} = Ns \times Nd \times Nn \tag{14}$$

Le vecteur des observations $\underline{Y}$ comporte alors Imax lignes.

La matrice de passage $\underline{W}$ comprend une première partie $W^{\mu}$ comportant des premiers termes notés $W^{\mu}$ (I, m) et une deuxième partie $W^D$ comportant des deuxièmes termes notés $W^D(I,m)$. Cette matrice est appelée matrice de sensibilité des mesures Y aux propriétés optiques X.

Les premiers termes $W^{\mu}(I,m)$ vérifient l'équation suivante :

$$W^{\mu}(I,m) = -\sum_{i+j+k=n} A_{sd}^{(p,k)}.G_s^{1(p,i)}(\vec{r}_m).G_d^{1(p,j)}(\vec{r}_m).V_m \tag{15}$$

et les deuxièmes termes $W^D(I,m)$ vérifient l'équation suivante

$$W^D(I,m) = -\sum_{i+j+k=n} A_{sd}^{(p,k)}.\vec{\nabla} G_s^{1(p,i)}(\vec{r}_m).\vec{\nabla} G_d^{1(p,j)}(\vec{r}_m).V_m \tag{16}$$

où $V_m$ représente un élément de volume entourant la maille m, tel que le volume de la cellule de Voronoi. Autrement dit, $V_m$ représente le volume du voxel m.

Selon ce mode de réalisation, au cours de chaque itération, les transformées de Mellin Laplace, de différents ordres, de $G_s^1(t)$ et de $G_d^1(t)$ sont déterminées, notamment en utilisant l'expression suivante :

$$-\nabla D(\vec{r})\nabla G_s^{1(p,n)}(\vec{r}) + \left(\mu_a(\vec{r}) + \frac{p}{c}\right)G_s^{1(p,n)}(\vec{r}) = \begin{cases} \delta(r) \ \text{si } n = 0 \\ \dfrac{p}{c} G_s^{1(p,n-1)}(\vec{r}) \ \text{si } n > 0 \end{cases} \tag{17}$$

Ainsi, lors de chaque itération, on détermine $G_s^{1(p,n=0)}$ puis on détermine les transformées d'ordre supérieur de façon itérative, en utilisant l'expression ci-dessus lorsque n > 0. Lors de la première itération, on utilise D = D° et $\mu_a = \mu_a^0$, et lors des itérations suivantes, on utilise D = D$^1$ et $\mu_a = \mu_a^1$. Le même raisonnement s'applique naturellement pour la détermination des transformées de Mellin Laplace de la fonction $G_d^1(t)$.

La matrice de passage $\underline{W}$ comporte alors Imax lignes et 2M colonnes.

Chaque terme $W^\mu(I,m)$, $\overline{W^D}(I,m)$ de la matrice de passage $\underline{W}$ est déterminé par modélisation, à chaque itération, en fonction des propriétés optiques déterminées lors de l'itération précédente, ou lors de la première itération, en fonction de propriétés optiques initialisées par l'opérateur. Cette initialisation est réalisée en considérant par exemple un milieu 12 homogène.

Le vecteur des inconnues $\underline{X}$ comporte 2M lignes et une colonne, et contient les inconnues $\mu_a(m)$ et D(m) pour chacun des M voxels. Les M premières lignes correspondent aux inconnues $\mu_a(m)$ et les M lignes suivantes du vecteur $\underline{X}$ correspondent aux inconnues D(m).

L'inversion de la matrice de passage $\underline{W}$ est réalisée selon des algorithmes d'inversion bien connus de l'homme du métier, tels qu'un algorithme de descente de gradient, une technique de reconstruction algébrique (ART), une décomposition en valeurs singulières (SVD), ou encore une méthode des gradients conjugués. Le processus s'arrête lorsqu'un critère de convergence est atteint, par exemple lorsque la distance entre deux vecteurs $\underline{X}$ successifs est inférieure à un seuil prédéterminé.

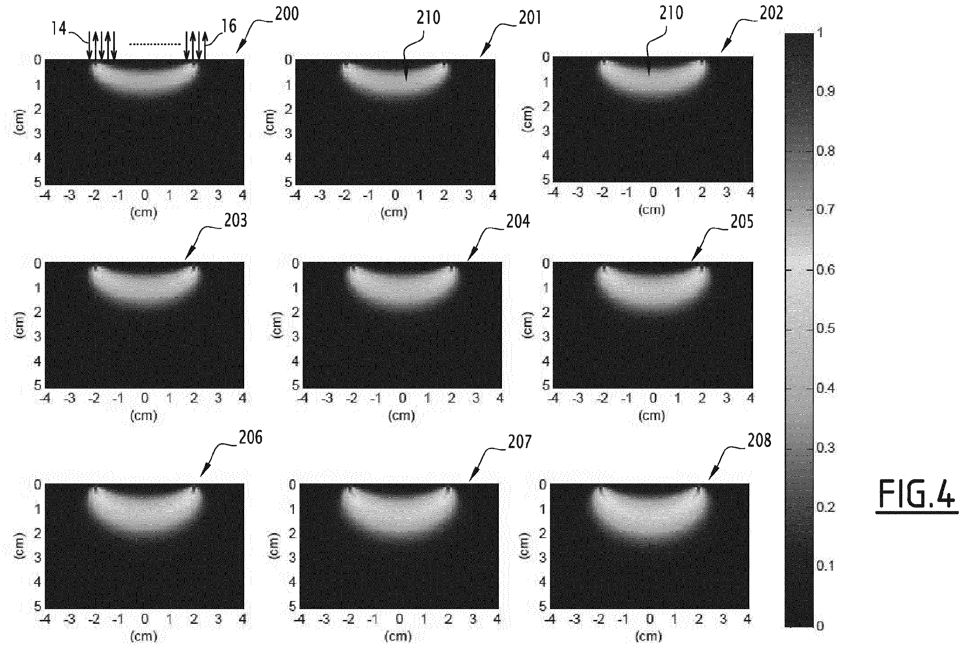

La figure 4 est un ensemble d'images 200 à 208 représentant une sensibilité $J_{sd}^{(p,n)}$ définie par l'équation :

$$J_{sd}^{(p,n)}(\vec{r}) = \frac{\partial Y_{sd}^{(p,n)}(\vec{r})}{\partial \mu(\vec{r})} \qquad (18)$$

pour les transformées de Mellin-Laplace de l'ensemble des distributions temporelles corrigées $Y_{sd}^{(p,n)}$, avec 21 sources 14 et 21 détecteurs 16, symbolisés respectivement par une flèche descendante et une flèche montante. L'espace entre une source 14 et un détecteur 16 successifs est d'environ un millimètre.

Chaque image 200 à 208 correspondant à une valeur respective et croissante de l'ordre n comprise entre 0 et 8, l'image 200 étant associée à l'ordre 0 et l'image 208 étant associée à l'ordre 8. Sur chaque image 200 à 208, l'ensemble des distributions temporelles corrigées $Y_{sd}^{(p,n)}$ correspond à une zone claire 210 en forme de banane.

L'homme du métier remarquera alors que plus la valeur de l'ordre n est élevée, meilleure est la sensibilité dans une zone profonde du milieu 12, par exemple à une profondeur supérieure à un centimètre. Autrement dit, plus la valeur de l'ordre n est élevée, plus le système de reconstruction 10 suivant le procédé selon l'invention est apte à observer la zone profonde du milieu 12.

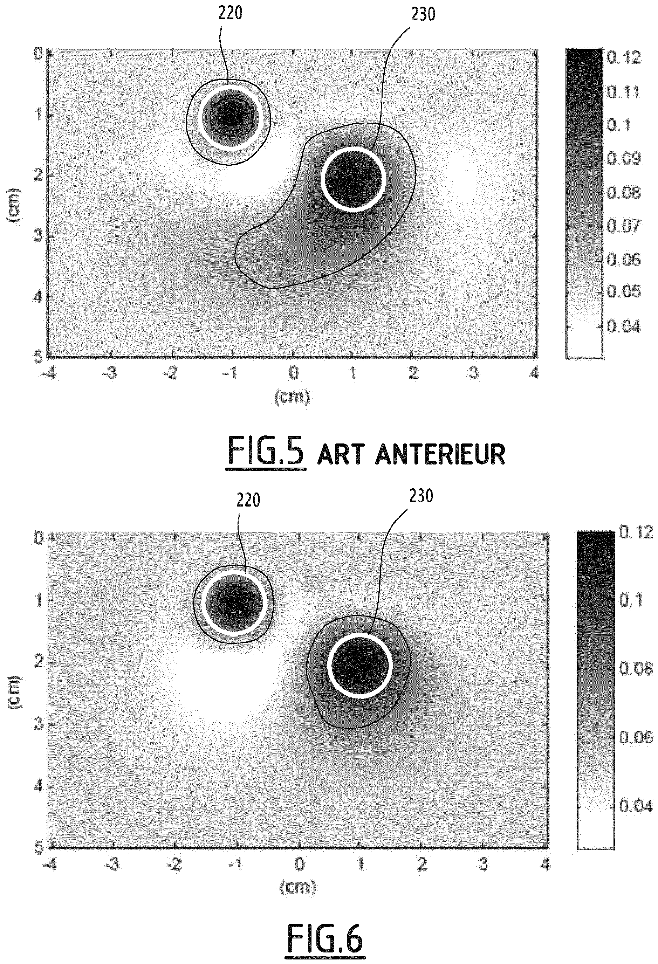

Le système de reconstruction 10 et le procédé de reconstruction selon l'invention permettent de localiser plus précisément des absorbeurs disposés en profondeur qu'un système d'imagerie et un procédé de reconstruction de l'état de la technique, comme le montrent les figures 5 et 6. La figure 5 est une vue d'une image d'un milieu comportant deux absorbeurs disposés à une profondeur de 1 cm, et respectivement de 2 cm, repérés par les cercles 220, et respectivement 230, reconstruite avec un système d'imagerie et un procédé de reconstruction de l'état de la technique, alors que la figure 6 est une vue d'une image du même milieu reconstruite avec un système d'imagerie et un procédé de reconstruction selon l'invention. En comparant les figures 5 et 6, l'homme du métier notera que les absorbeurs sont localisés avec une meilleure précision dans le cas du système d'imagerie et du procédé de reconstruction selon l'invention, notamment pour l'absorbeur disposé à 2 cm de profondeur.

La reconstruction des propriétés optiques à l'aide de la combinaison d'une pluralité de transformées de Mellin-Laplace de la grandeur comportant la distribution élaborée, en particulier à l'aide de la combinaison des $n_{max}+1$ transformées de Mellin-Laplace $Y_{sd}^{(p,n)}$, permet de converger plus rapidement vers la solution, afin de réduire le temps de traitement nécessaire à la reconstruction des propriétés optiques du milieu.

Un autre avantage d'une telle combinaison est qu'elle permet de prendre en compte les photons diffusés détectés peu après l'impulsion lumineuse, c'est-à-dire jusqu'à 1 ou 2 ns après l'impulsion lumineuse, également appelés photons prompts, en utilisant une transformée de Mellin Laplace aux faibles ordres, telles que des valeurs de l'ordre n comprises entre 0 et 5, tout en prenant également en compte les photons dits retardés, c'est-à-dire détectés quelques ns, voire plus de 5ns, après l'impulsion lumineuse. En utilisant des transformées de Mellin Laplace de différents ordres pour réaliser la reconstruction, on prend en compte à la fois les photons dits prompts et les photons dits retardés, ces derniers provenant généralement de profondeurs plus importantes. Il est donc avantageux d'effectuer la reconstruction en utilisant des données d'entrées combinant à la fois des transformées de Mellin Laplace de différents ordres. Par données d'entrée, on entend les données observées, c'est-à-dire les termes constituant le vecteur des observations $\underline{Y}$.

On décrit à présent une variante selon laquelle on détermine expérimentalement la réponse instrument $IRF_{sd}(t)$ correspondant chaque couple source-détecteur.

La reconstruction s'effectue selon un procédé itératif, en réalisant une mesure $B_{sd}(t)$ pour un ou différents couple(s) source-détecteur.

Pour chaque couple source-détecteur, on obtient alors l'équation suivante :

$$B_{sd}(t) - B_{sd}^1(t) = -S_s(t) * \left( \int G_s^1(\vec{r},t) * \delta\mu_a(\vec{r},t) * G_d^1(\vec{r},t) d\vec{r} + \int \vec{\nabla} G_s^1(\vec{r},t) * \delta D(\vec{r},t) * \vec{\nabla} G_d^1(\vec{r},t) d\vec{r} \right) * D_d(t) \quad (19)$$

où :

- $B_{sd}^1$ représente une mesure modélisée en considérant que le milieu présente les coefficients d'absorption $\mu_a^1(r)$ et de diffusion $D_a^1(r)$ déterminés lors de l'itération précédente, les valeurs $\mu_a^1(r)$ et $D_a^1(r)$ étant initialisées lors de la première itération par une première estimation réalisée par l'opérateur,

- les coefficients $\delta\mu_a(\vec{r}, t)$ et $\delta D(\vec{r}, t)$ représentent la différence entre les propriétés optiques du milieu ($\mu_a$, D) et les propriétés optiques ($\mu_a^1$, $D^1$) initiales, ou bien résultant de l'itération précédente.

De manière analogue au mode de réalisation précédent, l'objectif est de minimiser $\delta u_a(\vec{r}, t)$ et $\delta D(\vec{r}, t)$.

Si $B_{sd}(t)$ est une mesure réalisée sur le milieu à caractériser MC pour un couple source détecteur sd donné, on détermine les transformées de Mellin Laplace d'une fonction $Y_{sd}$, ces transformées se présentent sous la forme :

$$Y_{s,d}^{(p,n)} = \frac{B_{s,d}^{(p,n)} - \sum_{i=0}^{n-1} IRF_{s,d}^{(p,n-i)} B_{s,d}^{(p,i)}}{I_{s,d}^{(p,0)}} \quad (20)$$

Ainsi, en fonction des transformées de Mellin-Laplace de la réponse instrument $IRF_{s,d}^{(p,k)}$, avec $0 \le k \le n$, et de la mesure $B_{s,d}^{(p,k)}$ avec $0 \le k \le n$, on détermine $Y_{s,d}^{(p,n)}$ :

$$Y_{s,d}^{(p,n)} = \left( \int_\Omega \sum_{j+k=n} G_s^{1(p,j)}(\vec{r}).\delta\mu_a(\vec{r}).G_d^{1(p,k)}(\vec{r}) d\vec{r} + \int_\Omega \sum_{j+k=n} G_s^{1(p,j)}(\vec{r}).\delta D(\vec{r}).G_d^{1(p,k)}(\vec{r}) d\vec{r} \right) \quad (21)$$

Après discrétisation du milieu en M voxels m, cette expression devient :

$$Y_{s,d}^{(p,n)} = \sum_{m=1}^M \left( \sum_{j+k=n} G_s^{1(p,j)}(\vec{r}_m).\delta\mu_a(\vec{r}_m).G_d^{1(p,k)}(\vec{r}_m) V_m + \sum_{j+k=n} G_s^{1(p,j)}(\vec{r}_m).\delta D(\vec{r}_m).G_d^{1(p,k)}(\vec{r}_m) V_m \right) \quad (22)$$

On met alors en oeuvre un algorithme d'inversion itératif pour définir les propriétés optiques minimisant les vecteurs $\delta\mu_a(\vec{r}_m)$ et $\delta D(\vec{r}_m)$, à partir d'une pluralité de transformées de Mellin Laplace $Y_{s,d}^{(p,n)}$, avec des ordres différents, obtenues à l'aide de mesures réalisées avec une pluralité de couples sources détecteurs.

Selon un deuxième mode de réalisation, on utilise la transformée de Mellin Laplace pour établir une carte de fluorescence $F(\vec{r})$, avec $F(\vec{r}) = \mu_a(\vec{r})\eta$, $\eta$ étant le rendement de fluorescence du fluorophore localisé dans le volume $d\vec{r}$. On définit une fonction de réponse du fluorophore $F(\vec{r},t)$, telle que

$$F(\vec{r},t) = F(\vec{r})\exp(-\frac{t}{\tau}) \quad (23)$$

avec $\tau$ désignant le temps de vie du fluorophore.

A l'aide d'une source d'excitation s et d'un détecteur d tels que ceux définis dans le premier mode de réalisation, on réalise une mesure temporelle $B_{sd}(t)$ sur le milieu à caractériser MC.

Cette mesure s'écrit :

$$B_{sd}(t) = IRF_{sd}(t) * \int_{\Omega} G_s(\vec{r},t) * F(\vec{r},t) * G_d(\vec{r},t).d\vec{r} \qquad (24)$$

où $G_s(\vec{r},t)$ et $G_d(\vec{r},t)$ sont des fonctions de Green représentant la densité de photons respectivement entre la source s et le point $\vec{r}$ du milieu, et entre le point $\vec{r}$ du milieu et le détecteur d.
Comme

$$\left[\frac{\exp(-t/\tau)}{\tau}\right]^{(p,n)} = \frac{p^n}{n!}\int_0^{\infty} t^n \frac{\exp(-t/\tau)}{\tau}.dt = (p\tau)^n \qquad (25)$$

nous obtenons :

$$F^{(p,n)}(\vec{r}) = F(\vec{r})(p\tau)^n \qquad (26)$$

La transformée de Mellin-Laplace de la distribution temporelle mesurée $B_{sd}$ est donc :

$$B_{sd}^{(p,n)} = \sum_{i+j+k+l=n} IRF_{sd}^{(p,i)}.(p\tau)^j.\int_{\Omega} G_s^{(p,k)}(\vec{r}).F(\vec{r}).G_{sd}^{(p,l)}(\vec{r}).d\vec{r} \qquad (27)$$

Après discrétisation du milieu en M voxels m, on obtient :

$$B_{sd}^{(p,n)} = \sum_{i+j+k+l=n} IRF_{sd}^{(p,i)}.(p\tau)^j \sum_{m=1}^{M} \left( G_s^{(p,k)}(\vec{r}_m).F(\vec{r}_m).G_{sd}^{(p,l)}(\vec{r}_m)V_m \right). \qquad (28)$$

On inverse enfin ce système après discrétisation du milieu en voxels. On met alors en oeuvre un algorithme d'inversion, tel qu'un des algorithmes connus mentionnés précédemment, pour définir le vecteur $F(\vec{r}_m)$ correspondant

à la carte de fluorescence recherchée, à partir de la pluralité de transformées de Mellin Laplace $Y_{s,d}^{(p,n)}$, avec des ordres différents, obtenues à l'aide de mesures réalisées avec une pluralité de couples sources détecteurs sd.

De manière analogue au premier mode de réalisation, la combinaison de différentes transformées de Mellin Laplace d'une grandeur $Y_{s,d}^{(p,n)}$ correspondant à un couple source détecteur, et notamment le recours à différentes valeurs de l'ordre n, permet d'obtenir une meilleure prise en compte les contributions des photons prompts et des photons retardés, ces derniers provenant essentiellement de profondeurs plus élevées.

## Revendications

1. Procédé de reconstruction de propriétés optiques d'un milieu (12), à l'aide d'un système de reconstruction (10) comportant au moins une source de rayonnement (14, s) propre à éclairer le milieu (12) et au moins un détecteur (16, d) propre à recevoir un signal émis par le milieu (12),
le procédé comprenant les étapes suivantes :

   - l'éclairement (100) du milieu (12) par la ou chaque source de rayonnement (14, s),
   - la réception, par le ou chaque détecteur (16, d), d'un signal émis par le milieu (12), et
   - l'élaboration (110), pour au moins un couple source-détecteur (14, s, 16, d), d'une première distribution ($B_{sd}(t)$) du signal reçu par le détecteur (16, d) correspondant,
   - le calcul (130) de la transformée de Mellin-Laplace ($Y_{sd}^{(p,n)}$ ; $B_{sd}^{(p,n)}$, pour un ordre (n) et une variable (p) donnés, d'une grandeur ($Y_{sd}(t)$ ; $B_{sd}(t)$) comportant la première distribution ($B_{sd}(t)$), l'ordre (n) étant un nombre entier, la variable (p) étant un nombre réel, et
   - la reconstruction (140) de propriétés optiques du milieu (12) à l'aide de la transformée de Mellin-Laplace ($Y_{sd}^{(p,n)}$ ; $B_{sd}^{(p,n)}$ de ladite grandeur ($Y_{sd}(t)$ ; $B_{sd}(t)$),

**caractérisé en ce que** l'étape de calcul (130) comporte le calcul d'une pluralité de transformées de Mellin-Laplace $(Y_{sd}^{(p,n)}$ ; $B_{sd}^{(p,n)}$ de ladite grandeur pour des valeurs distinctes de l'ordre (n), et **en ce que** l'étape de reconstruction (140) est effectuée à partir d'une combinaison de la pluralité de transformées de Mellin-Laplace $(Y_{sd}^{(p,n)}$; $B_{sd}^{(p,n)})$.

2. Procédé selon la revendication 1, dans lequel l'étape de calcul (130) comporte le calcul d'au moins une transformée de Mellin-Laplace $(Y_{sd}^{(p,n)n)}$ ; $B_{sd}^{(p,n)}$ de ladite grandeur pour une valeur de l'ordre (n) supérieure ou égale à 5, de préférence supérieure ou égale à 8.

3. Procédé selon la revendication 2, dans lequel l'étape de calcul (130) comporte le calcul de $n_{max}+1$ transformées de Mellin-Laplace $(Y_{sd}^{(p,n)}$ ; $B_{sd}^{(p,n)})$, l'ordre (n) prenant successivement toutes les valeurs comprises entre 0 et $n_{max}$, avec $n_{max}$ supérieur ou égal à 5, de préférence supérieur ou égal à 8.

4. Procédé selon l'une quelconque des revendications précédentes, dans lequel la valeur de la variable (p) est comprise entre 1 $ns^{-1}$ et 20 $ns^{-1}$.

5. Procédé selon l'une quelconque des revendications précédentes, dans lequel le procédé comprend en outre :

- la détermination, pour au moins un couple source-détecteur (14, s, 16, d), d'une première fonction $(G_{sd}(t))$ de modélisation d'un signal de diffusion de la lumière entre la source (14, s) et le détecteur (16, d) dans le milieu (12),
- l'élaboration, pour ledit au moins un couple source-détecteur (14, s, 16, d), d'une deuxième distribution $(A_{sd}(t))$ d'un signal reçu par le détecteur (16, d) pour un milieu de référence (MR), le signal reçu étant émis par le milieu de référence (MR) suite à l'éclairement dudit milieu (MR) par la source (14, s), et
- la détermination, pour ledit au moins un couple source-détecteur (14, s, 16, d), d'une deuxième fonction $(G_{sd}^0(t))$ de modélisation d'un signal de diffusion de la lumière entre la source (14, s) et le détecteur (16, d) dans le milieu de référence (MR),

et dans lequel ladite grandeur $(Y_{sd}(t))$ dépend de la première distribution $(B_{sd}(t))$, de la première fonction de modélisation $(G_{sd}(t))$, de la deuxième distribution $(A_{sd}(t))$ et de la deuxième fonction de modélisation $(G_{sd}^0(t))$.

6. Procédé selon la revendication 5, dans lequel ladite grandeur $(Y_{sd}(t))$ est obtenue par soustraction du produit de la deuxième distribution $(A_{sd}(t))$ et de la première fonction de modélisation $(G_{sd}(t))$ du produit de la première distribution $(B_{sd}(t))$ et de la deuxième fonction de modélisation $(G_{sd}^0(t))$.

7. Procédé selon la revendication 5, dans lequel ladite grandeur $(Y_{sd}(t))$ est le ratio entre le produit de la première distribution $(B_{sd}(t))$ et de la deuxième fonction de modélisation $(G_{sd}^0(t))$ et le produit de la deuxième distribution $(A_{sd}(t))$ et de la première fonction de modélisation $(G_{sd}(t))$.

8. Procédé selon l'une quelconque des revendications précédentes, dans lequel la ou chaque source de rayonnement (14, s) comporte une source de lumière puisée.

9. Procédé selon l'une quelconque des revendications précédentes, dans lequel le ou chaque détecteur (16, d) est un détecteur résolu en temps.

10. Procédé selon l'une quelconque des revendications précédentes, dans lequel les propriétés optiques comprennent au moins un élément parmi le groupe consistant en :

- les propriétés d'absorption de la lumière, caractérisées notamment par le coefficient d'absorption $(\mu_a)$,
- les propriétés de diffusion, caractérisées notamment par le coefficient de diffusion réduit $(\mu'_s)$ ou par le coefficient de diffusion (D), et
- les propriétés de fluorescence, caractérisées notamment par une fonction de réponse (F) d'un fluorophore, ou par une concentration (c) du fluorophore, ou encore par une grandeur fonction d'une quantité (q) du fluorophore.

11. Système (10) de reconstruction de propriétés optiques d'un milieu (12), comprenant :

- au moins une source (14, s) de rayonnement propre à éclairer le milieu (12),
- au moins un détecteur (16, d) propre à recevoir un signal émis par le milieu (12),
- des moyens (42) d'élaboration, pour au moins un couple source-détecteur (14, s, 16, d), d'une distribution

($B_{sd}(t)$) du signal reçu par le détecteur (16, d) correspondant,
- des moyens (44) de calcul de la transformée de Mellin-Laplace ($Y_{sd}^{(p,n)}$ ; $B_{sd}^{(p,n)}$), pour un ordre (n) et une variable (p) donnés, d'une grandeur ($Y_{sd}(t)$ ; $B_{sd}(t)$) comportant la distribution ($B_{sd}(t)$), l'ordre (n) étant un nombre entier, la variable (p) étant un nombre réel, et
- des moyens (46) de reconstruction de propriétés optiques du milieu (12) à l'aide de la transformée de Mellin-Laplace ($Y_{sd}^{(p,n)}$ ; $B_{sd}^{(p,n)}$) de ladite grandeur ($Y_{sd}(t)$ ; $B_{sd}(t)$),

**caractérisé en ce que** les moyens de calcul (44) sont propres à calculer une pluralité de transformées de Mellin-Laplace ($Y_{sd}^{(p,n)}$ ; $B_{sd}^{(p,n)}$) de ladite grandeur pour des valeurs distinctes de l'ordre (n), et **en ce que** les moyens de reconstruction (46) sont propres à reconstruire les propriétés optiques du milieu (12) à partir d'une combinaison de la pluralité de transformées de Mellin-Laplace ($Y_{sd}^{(p,n),n}$ ; $B_{sd}^{(p,n)}$).

**12.** Système (10) selon la revendication 11, dans lequel les moyens de calcul (44) sont propres à calculer au moins une transformée de Mellin-Laplace ($Y_{sd}^{(p,n)}$ ; $B_{sd}^{(p,n)}$) de ladite grandeur pour une valeur de l'ordre (n) supérieure ou égale à 5, de préférence supérieure ou égale à 8.

**Patentansprüche**

**1.** Verfahren zum Rekonstruieren von optischen Eigenschaften eines Mediums (12) mit Hilfe eines Systems zum Rekonstruieren (10), welches aufweist mindestens eine Strahlungsquelle (14, s), die imstande ist, das Medium (12) zu bestrahlen, und mindestens einen Detektor (16, d), welcher imstande ist, ein Signal zu empfangen, welches von dem Medium (12) gesendet wird,
wobei das Verfahren die folgenden Schritte aufweist:

- das Bestrahlen (100) des Mediums (12) mittels der oder jeder Strahlungsquelle (14, s),
- das Empfangen mittels des oder jedes Detektors (16, d) eines Signals, welches von dem Medium (12) gesendet wird, und
- das Erstellen (110) für mindestens ein Quelle-Detektor-Paar (14, s, 16, d) von einer ersten Verteilung ($B_{sd}(t)$) des Signals, welches mittels des korrespondierenden Detektors (16, d) empfangen wird,
- das Berechnen (130) der Mellin-Laplace-Transformierten ($Y_{sd}^{(p,n)}$; $B_{sd}^{(p,n)}$) für eine gegebene Ordnung (n) und eine gegebene Variable (p) von einer Größe ($Y_{sd}(t)$; $B_{sd}(t)$), welche die erste Verteilung ($B_{sd}(t)$) aufweist, wobei die Ordnung (n) eine ganze Zahl ist, wobei die Variable (p) eine reelle Zahl ist, und
- das Rekonstruieren (140) von optischen Eigenschaften des Mediums (12) mit Hilfe der Mellin-Laplace-Transformierten ($Y_{sd}^{(p,n)}$; $B_{sd}^{(p,n)}$) der besagten Größe ($Y_{sd}(t)$; $B_{sd}(t)$),

**dadurch gekennzeichnet, dass** der Schritt des Berechnens (130) aufweist das Berechnen von einer Mehrzahl von Mellin-Laplace-Transformierten ($Y_{sd}^{(p,n)}$; $B_{sd}^{(p,n)}$) der besagten Größe für verschiedene Werte der Ordnung (n) und dadurch, dass der Schritt des Rekonstruierens (140) durchgeführt wird ausgehend von einer Kombination der Mehrzahl von Mellin-Laplace-Transformierten ($Y_{sd}^{(p,n)}$; $B_{sd}^{(p,n)}$).

**2.** Verfahren gemäß Anspruch 1, wobei der Schritt des Berechnens (130) aufweist das Berechnen von mindestens einer Mellin-Laplace-Transformierten ($Y_{sd}^{(p,n)}$; $B_{sd}^{(p,n)}$) der besagten Größe für einen Wert der Ordnung (n), der größer oder gleich 5, vorzugsweise größer oder gleich 8 ist.

**3.** Verfahren gemäß Anspruch 2, wobei der Schritt des Berechnens (130) aufweist das Berechnen von $n_{max}$+1 Mellin-Laplace-Transformierten ($Y_{sd}^{(p,n)}$; $B_{sd}^{(p,n)}$), wobei die Ordnung (n) sukzessive alle Werte annimmt, die zwischen 0 und $n_{max}$ enthalten sind, mit $n_{max}$ größer oder gleich 5, vorzugsweise größer oder gleich 8.

**4.** Verfahren gemäß irgendeinem der vorherigen Ansprüche, wobei der Wert der Variablen (p) zwischen 1 ns$^{-1}$ und 20 ns$^{-1}$ enthalten ist.

**5.** Verfahren gemäß irgendeinem der vorherigen Ansprüche, wobei das Verfahren ferner aufweist:

- das Ermitteln für mindestens ein Quelle-Detektor-Paar (14, s, 16, d) von einer ersten Funktion ($G_{sd}(t)$) zur Modellierung eines Signals der Diffusion des Lichtes zwischen der Quelle (14, s) und dem Detektor (16, d) in dem Medium (12),
- das Erstellen für das besagte mindestens eine Quelle-Detektor-Paar (14, s, 16, d) von einer zweiten Verteilung

($A_{sd}(t)$) eines Signals, welches mittels des Detektors (16, d) empfangen wird, für ein Referenz-Medium (MR), wobei das empfangene Signal von dem Referenz-Medium (MR) gesendet wird in Folge des Bestrahlens des besagten Mediums (MR) mittels der Quelle (14, s), und

- das Ermitteln für das besagte mindestens eine Quelle-Detektor-Paar (14, s, 16, d) von einer zweiten Funktion ($G_{sd}^0(t)$) zur Modellierung eines Signals der Diffusion des Lichtes zwischen der Quelle (14, s) und dem Detektor (16, d) in dem Referenz-Medium (MR),

und wobei die besagte Größe ($Y_{sd}(t)$) abhängt von der ersten Verteilung ($B_{sd}(t)$), von der ersten Funktion zur Modellierung ($G_{sd}(t)$), von der zweiten Verteilung ($A_{sd}(t)$) und von der zweiten Funktion zur Modellierung ($G_{sd}^0(t)$).

6. Verfahren gemäß Anspruch 5, wobei die besagte Größe ($Y_{sd}(t)$) erlangt wird mittels Subtrahierens des Produkts der zweiten Verteilung ($A_{sd}(t)$) und der ersten Funktion zur Modellierung ($G_{sd}(t)$) von dem Produkt der ersten Verteilung ($B_{sd}(t)$) und der zweiten Funktion zur Modellierung ($G_{sd}^0(t)$).

7. Verfahren gemäß Anspruch 5, wobei die besagte Größe ($Y_{sd}(t)$) das Verhältnis ist zwischen dem Produkt der ersten Verteilung ($B_{sd}(t)$) und der zweiten Funktion zur Modellierung ($G_{sd}^0(t)$) und dem Produkt der zweiten Verteilung ($A_{sd}(t)$) und der ersten Funktion zur Modellierung ($G_{sd}(t)$).

8. Verfahren gemäß irgendeinem der vorherigen Ansprüche, wobei die oder jede Strahlungsquelle (14, s) eine Quelle gepulsten Lichtes aufweist.

9. Verfahren gemäß irgendeinem der vorherigen Ansprüche, wobei der oder jeder Detektor (16, d) ein zeitaufgelöster Detektor ist.

10. Verfahren gemäß irgendeinem der vorherigen Ansprüche, wobei die optischen Eigenschaften mindestens ein Element aus der Gruppe aufweisen, die besteht aus:

- den Absorptions-Eigenschaften des Lichtes, die insbesondere charakterisiert sind durch den Absorptions-Koeffizienten ($\mu_a$),
- den Diffusions-Eigenschaften, die insbesondere charakterisiert sind durch den reduzierten Diffusions-Koeffizienten ($\mu'_s$) oder durch den Diffusions-Koeffizienten (D), und
- den Fluoreszenz-Eigenschaften, die insbesondere charakterisiert sind durch eine Antwortfunktion (F) eines Fluorophors oder durch eine Konzentration (c) des Fluorophors oder durch eine Größe, die abhängig von einer Menge (q) des Fluorophors ist.

11. System (10) zum Rekonstruieren von optischen Eigenschaften eines Mediums (12), welches aufweist:

- mindestens eine Quelle (14, s) von Strahlung, die imstande ist, das Medium (12) zu bestrahlen,
- mindestens einen Detektor (16, d), der imstande ist, ein Signal zu empfangen, welches von dem Medium (12) gesendet wird,
- Mittel (42) zum Erstellen für mindestens ein Quelle-Detektor-Paar (14, s, 16, d) von einer Verteilung ($B_{sd}(t)$) des Signals, welches mittels des korrespondierenden Detektors (16, d) empfangen wird,
- Mittel (44) zum Berechnen der Mellin-Laplace-Transformierten ($Y_{sd}^{(p,n)}$; $B_{sd}^{(p,n)}$) für eine gegebene Ordnung (n) und eine gegebene Variable (p) von einer Größe ($Y_{sd}(t)$; $B_{sd}(t)$), welche die Verteilung ($B_{sd}(t)$) aufweist, wobei die Ordnung (n) eine ganze Zahl ist, wobei die Variable (p) eine reelle Zahl ist, und
- Mittel (46) zum Rekonstruieren von optischen Eigenschaften des Mediums (12) mit Hilfe der Mellin-Laplace-Transformierten ($Y_{sd}^{(p,n)}$; $B_{sd}^{(p,n)}$) der besagten Größe ($Y_{sd}(t)$; $B_{sd}(t)$),

dadurch gekennzeichnet, dass die Mittel zum Berechnen (44) imstande sind, eine Mehrzahl von Mellin-Laplace-Transformierten ($Y_{sd}^{(p,n)}$; $B_{sd}^{(p,n)}$) der besagten Größe für verschiedene Werte der Ordnung (n) zu berechnen, und dadurch, dass die Mittel zum Rekonstruieren (46) imstande sind, die optischen Eigenschaften des Mediums (12) zu rekonstruieren ausgehend von einer Kombination der Mehrzahl von Mellin-Laplace-Transformierten ($Y_{sd}^{(p,n)}$; $B_{sd}^{(p,n)}$).

12. System (10) gemäß Anspruch 11, wobei die Mittel zum Berechnen (44) imstande sind, mindestens eine Mellin-Laplace-Transformierte ($Y_{sd}^{(p,n)}$; $B_{sd}^{(p,n)}$) der besagten Größe für einen Wert der Ordnung (n), der größer oder gleich 5, vorzugsweise größer oder gleich 8 ist, zu berechnen.

**Claims**

1. A method for reconstructing the optical properties of a medium (12), using a reconstruction system (10) comprising at least one radiation source (14, s) capable of lighting the medium (12) and at least one detector (16, d) capable of receiving a signal transmitted by the medium (12),
the method including the following steps:

   - lighting (100) the medium (12) using the or each radiation source (14, s),
   - receiving, by the or each detector (16, d), the signal transmitted by the medium (12), and
   - processing (110), for at least one source-detector pair (14, s, 16, d), a first distribution ($B_{sd}(t)$) of the signal received by the corresponding detector (16, d),
   - computing (130) the Mellin-Laplace transform ($Y_{sd}^{(p,n)}$ ; $B_{sd}^{(p,n)}$), for a given order (n) and a given variable (p), of a magnitude ($Y_{sd}(t)$ ; $B_{sd}(t)$) comprising the first distribution ($B_{sd}(t)$), the order (n) being an integer, the variable (p) being a real number, and
   - reconstructing (140) optical properties of the medium (12) using the Mellin-Laplace transform ($Y_{sd}^{(p,n)}$ ; $B_{sd}^{(p,n)}$) of said magnitude ($Y_{sd}(t)$ ; $B_{sd}(t)$),

   **characterized in that** the computing step (130) comprises computing a plurality of Mellin-Laplace transforms ($Y_{sd}^{(p,n)}$ ; $B_{sd}^{(p,n)}$) of said magnitude for distinct values of the order (n), and **in that** the reconstructing step (140) is carried out from a combination of the plurality of Mellin-Laplace transforms ($Y_{sd}^{(p,n)}$ ; $B_{sd}^{(p,n)}$).

2. The method according to claim 1, wherein the computing step (130) comprises computing at least one Mellin-Laplace transform ($Y_{sd}^{(p,n)}$ ; $B_{sd}^{(p,n)}$) of said magnitude for an order (n) value greater than or equal to 5, preferably greater than or equal to 8.

3. The method according to claim 2, wherein the computing step (130) comprises the computing of $n_{max}+1$ Mellin-Laplace transforms ($Y_{sd}^{(p,n)}$; $B_{sd}^{(p,n)}$), the order (n) successively taking all values comprised between 0 and $n_{max}$, $n_{max}$ being greater than or equal to 5, preferably greater than or equal to 8.

4. The method according to any one of the preceding claims, wherein the value of the variable (p) is between 1 ns$^{-1}$ and 20 ns$^{-1}$.

5. The method according to any one of the preceding claims, wherein the method also includes:

   - determining, for at least one source-detector pair (14, s, 16, d), a first modeling function ($G_{sd}(t)$) of a diffusion signal of the light between the source (14, s) and the detector (16, d) in the medium (12),
   - processing, for said at least one source-detector pair (14, s, 16, d), a second distribution ($A_{sd}(t)$) of a signal received by the detector (16, d) for a reference medium (MR), the received signal being transmitted by the reference medium (MR) following the lighting of said medium (MR) by the source (14, s), and
   - determining, for said at least one source-detector pair (14, s, 16 d), a second modeling function ($G_{sd}^{0}(t)$) of a diffusion signal of the light between the source (14, s) and the detector (16, d) in the reference medium (MR),

   and wherein said magnitude ($Y_{sd}(t)$) depends on the first distribution ($B_{sd}(t)$), the first modeling function ($G_{sd}(t)$), the second distribution ($A_{sd}(t)$) and the second modeling function ($G_{sd}^{0}(t)$).

6. The method according to claim 5, wherein said magnitude ($Y_{sd}(t)$) is obtained by subtracting the product of the second distribution ($A_{sd}(t)$) and the first modeling function ($G_{sd}(t)$) from the product of the first distribution ($B_{sd}(t)$) and the second modeling function ($G_{sd}^{0}(t)$).

7. The method according to claim 5, wherein said magnitude ($Y_{sd}(t)$) is the ratio between the product of the first distribution ($B_{sd}(t)$) and the second modeling function ($G_{sd}^{0}(t)$) and the product of the second distribution ($A_{sd}(t)$) and the first modeling function ($G_{sd}(t)$).

8. The method according to any one of the preceding claims, wherein the or each radiation source (14, s) comprises a pulsed light source.

9. The method according to any one of the preceding claims, wherein the or each detector (16, d) is a time-resolved detector.

10. The method according to any one of the preceding claims, wherein the optical properties include at least one element from amongst the group consisting of:

- the light absorption properties, in particular **characterized by** the absorption coefficient ($\mu_a$),
- the diffusion properties, in particular **characterized by** the reduced diffusion coefficient ($\mu'_s$) or the diffusion coefficient (D), and
- the fluorescence properties, in particular **characterized by** a response function (F) of a fluorophore, or by a concentration (c) of the fluorophore, or by a magnitude depending on the quantity (q) of the fluorophore.

11. A system (10) for reconstructing optical properties of a medium (12), including:

- at least one radiation source (14, s) capable of lighting the medium (12),
- at least one detector (16, d) capable of receiving a signal transmitted by the medium (12),
- processing means (42) for processing a distribution ($B_{sd}(t)$) of the signal received by the corresponding detector (16, d) for at least one source-detector pair (14, s, 16, d),
- computing means (44) for computing the Mellin-Laplace transform ($Y_{sd}^{(p,n)}$ ; $B_{sd}^{(p,n)}$), for a given order (n) and a given variable (p), of a magnitude ($Y_{sd}(t)$; $B_{sd}(t)$) comprising the distribution ($B_{sd}(t)$), the order (n) being an integer, the variable (p) being a real number, and
- reconstructing means (46) for reconstructing the optical properties of the medium (12) using the Mellin-Laplace transform ($Y_{sd}^{(p,n)}$ ; $B_{sd}^{(p,n)}$) of said magnitude ($Y_{sd}(t)$ ; $B_{sd}(t)$),

**characterized in that** the computing means (44) are capable of computing the plurality of Mellin-Laplace transforms ($Y_{sd}^{(p,n)}$ ; $B_{sd}^{(p,n)}$) of said magnitude for distinct values of the order (n), and **in that** the reconstructing means (46) are capable of reconstructing the optical properties of the medium (12) from a combination of the plurality of Mellin-Laplace transforms ($Y_{sd}^{(p,n)}$ ; $B_{sd}^{(p,n)}$).

12. The system (10) according to claim 11, wherein the computing means (44) are capable of computing at least one Mellin-Laplace transform ($Y_{sd}^{(p,n)}$; $B_{sd}^{(p,n)}$) of said magnitude for an order (n) value greater than or equal to 5, preferably greater than or equal to 8.

## FIG.1

EP 2 605 002 B1

FIG.2

Eclairement d'un milieu à l'aide de chaque source de rayonnement — 100

Elaboration d'une distribution pour le milieu et pour chaque couple source-détecteur — 110

Prise en compte de la réponse instrument — 120

Calcul des transformées de Mellin-Laplace, pour tous les ordres compris entre 0 et $n_{max}$, d'une grandeur comportant la distribution — 130

Reconstruction de propriétés optiques du milieu — 140

FIG.3

FIG.4

EP 2 605 002 B1

## FIG.5 ART ANTERIEUR

## FIG.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1884765 A1

- FR 2950241 A1

**Littérature non-brevet citée dans la description**

- **S.R. ARRIDGE.** Optical tomography in medical imaging. *Inverse Problems,* 1999